# EUROPÄISCHE PATENTSCHRIFT

(11) **EP 3 634 379 B1**
(45) Veröffentlichungstag und Bekanntmachung des Hinweises auf die Patenterteilung: **28.08.2024**
(21) Anmeldenummer: 18731023.0
(22) Anmeldetag: 07.06.2018
(51) Int. Cl.: A61P 11/14, A61P 25/24, A61P 25/28, A61P 29/00, A61K 9/00, A61K 47/32, A61K 9/12, A61K 9/70, A61K 31/00

(54) **SCHNELL ZERFALLENDE SCHAUMWAFER MIT HOHEM FLÄCHENGEWICHT**
QUICKLY DISINTEGRATING FOAM WAFER WITH HIGH MASS PER UNIT AREA
PASTILLE EN MOUSSE À DÉCOMPOSITION RAPIDE, PRÉSENTANT UN POIDS SURFACIQUE ÉLEVÉ

(30) Priorität: 07.06.2017 DE 102017112527
(43) Veröffentlichungstag der Anmeldung: 15.04.2020
(73) Patentinhaber: LTS Lohmann Therapie-Systeme AG, 56626 Andernach (DE)
(72) Erfinder: MÜLLER, Markus, 53840 Troisdorf (DE); BAUER, Marius, 56626 Andernach (DE); LINN, Michael, 55596 Waldböckelheim (DE)
(74) Vertreter: Meissner Bolte Partnerschaft mbB
(86) Internationale Anmeldenummer: PCT/EP2018/065008
(87) Internationale Veröffentlichungsnummer: WO 2018/224591

(56) Entgegenhaltungen:
- EP-A2- 0 643 963
- WO-A2-02/02085
- WO-A2-02/066016
- DE-A1- 102005 058 569
- US-A- 6 090 401

## Beschreibung

Die Erfindung betrifft eine flächenförmige, in wässriger Umgebung sich auflösende oder zersetzende Darreichungsform zur Freisetzung von Wirkstoffen, die mittels der Darreichungsform insbesondere oral verabreicht werden können, und die eine Matrix auf Basis von wasserlöslichen Polymeren als Grundsubstanzen aufweisen, wie in den Patentansprüchen definiert.

Insbesondere betrifft die Erfindung Darreichungsformen der genannten Art, welche in Form von Wafern gestaltet sind. Ferner betrifft die Erfindung Verfahren zur Herstellung solcher Darreichungsformen.

### Stand der Technik

Zur Verabreichung von Wirkstoffen über die Mundschleimhaut werden üblicherweise Buccal- oder Sublingualtabletten verwendet, die den Wirkstoff im Mundraum freisetzen. Die Resorption des Wirkstoffs über die Mundschleimhaut bietet gegenüber anderen peroralen Darreichungsformen einige Vorteile, beispielsweise, dass Medikamente oral auch an Patienten mit Schluckbeschwerden verabreicht werden können, dass der Wirkungseintritt aufgrund einer Umgehung der Magen-Darm-Passage rasch erfolgt, und dass die Wirkstoffausnutzung hoch ist.

Als alternative Darreichungsform zu den bekannten Buccal- und Sublingualtabletten sind flächenförmige, oblatenartige Darreichungsformen bekannt, die auch als Wafer bezeichnet werden.

So beschreibt beispielsweise die US 5,529,782 ein schnell lösliches Filmprodukt aus löslichem Polymermaterial oder komplexen Polysacchariden, das vornehmlich zur Verabreichung von Kontrazeptiva eingesetzt wird. Das Filmprodukt soll eine Dicke von 3 bis 4,5 mm aufweisen und seine Löslichkeit soll so einstellbar sein, dass es sich innerhalb von 5 bis 60 Sekunden nach Verabreichung aufgelöst hat. Das Filmprodukt kann auch in Form eines Laminats vorliegen, welches mit Gas aufgeschäumte Hohlräume aufweist.

Aus der EP 0450 141 B1 ist ein Trägermaterial zur Verabreichung von Arzneimitteln bekannt, welches sich bei Kontakt mit Speichel schnell auflöst. Bei diesem Trägermaterial handelt es sich um einen porösen, dehydratisierten, skelettartigen Trägerstoff, insbesondere auf Basis von Proteinen und Polysacchariden. Die durch Dehydratisierung erzeugten Hohlräume werden für das Einbringen von flüssigen Wirkstoffen genutzt.

Die WO 98/26764 beschreibt eine bei Kontakt mit Flüssigkeit schnell zerfallende, wirkstoffhaltige und folienförmige Darreichungsform, bei der eine fettlösliche Phase in Form von flüssigen Tröpfchen in einer äußeren wasserlöslichen Phase verteilt ist.

In der WO 00/18365 wird eine essbare Folie vorgeschlagen, die schnell löslich sein soll, aber auch gut an der Mundschleimhaut haften kann, um antimikrobielle Substanzen abzugeben und die Zahl unerwünschter Mikroorganismen in der Mundflora verringert. Bei den antimikrobiellen Substanzen handelt es sich um ätherische Öle, die als lipophile Phase vorzugsweise mit Pullulan als Matrixmaterial in der wässrigen Phase vermischt werden.

Die US 2001/006677 offenbart folienförmige, aufschäumende und in Wasser lösliche oder quellfähige Darreichungsformen, die leicht an der Mundschleimhaut haften.

Die WO 02/02085 beschreibt schnell zerfallende Darreichungsformen zur Freisetzung von Wirkstoffen im Mundraum oder anderen Körperöffnungen, wobei die Darreichungsform eine Matrix aufweist, die zumindest ein wasserlösliches Polymer als Grundsubstanz enthält und die mit Hohlräumen versehen ist.

Die DE 10 2005 058 569 beschreibt ein Schaumwafer-Produkt zur Freisetzung von Wirkstoffen wie insbesondere Nikotin im Mundraum, das auf einem Polyvinylalkohol-Polyethylenglycol-Pfropfcopolymer beruht.

Das Wirkprinzip der vorstehend beschriebenen Wafer beruht darauf, dass sich die als Matrix für den jeweiligen Wirkstoff verwendeten Polymere bei Kontakt mit Wasser bzw. Speichel lösen und so der Wafer zerfällt, wobei die Wirkstoffe freigesetzt werden. Der Eintritt und der zeitliche Verlauf der Wirkstofffreisetzung hängt in hohem Maße von der Dicke des Wafers ab. Je dünner der Wafer, desto schneller geht der Zerfall in wässriger Umgebung vonstatten, da das Lösungsmittel schneller in die inneren Bereiche der Arzneiform vordringen kann. Andererseits müssen entsprechende Wafer eine gewisse Mindestdicke aufweisen, um ihre bestimmungsgemäße Funktion erfüllen zu können und ausreichend handhabbar zu sein. Zudem hängt die Dicke solcher Darreichungsformen von der Art und Menge des Wirkstoffes ab, den sie enthalten und freisetzen sollen. Mit zunehmender Dicke wird der Zerfall bzw. die Auflösung des Wafers entsprechend verlangsamt.

Insbesondere dickere Wafer, aber auch solche mit einer relativ geringen Dicke, neigen aufgrund ihrer flächigen, glatten Form bei verzögertem Zerfall dazu, am Gaumen oder an anderen Schleimhautoberflächen des Mundraums anzuhaften und festzukleben. Dies wird unter anderem durch die sich oberflächlich lösenden Polymerschichten bedingt, welche einen klebrigen und matschigen Film bilden. Dies hat dazu geführt, dass Wafersysteme heute in der Regel mit Wirkstoffen formuliert werden, von denen nur geringe Mengen eingesetzt werden müssen, da so größere Dicken, die durch große Wirkstoffmengen bedingt sind, vermieden werden können. Aufgrund der für solche Filme erforderlichen physikochemischen Eigenschaften (z.B. ausreichende Festigkeit) ist man für Wirkstoffe, die in größeren Mengen verabreicht werden müssen, heute daher immer noch auf konventionelle Verabreichungssysteme wie Tabletten angewiesen.

Ein weiteres Problem bei dickeren Wafern besteht in der verzögerten Freisetzung des Wirkstoffs oder von anderen Bestandteilen, wie insbesondere geschmacksmaskierenden Mitteln. Wenn diese in Form von unlöslichen oder schwerlöslichen Feststoffen vorliegen, verbleiben diese Feststoffe durch das langsamere Auslöseverhalten länger im Mund, was dort als unangenehm wahrgenommen werden kann. Um das durch Wafer hervorgerufene Empfinden im Mundraum zu verbessern, wurde in der vorstehend erwähnten WO 02/02085 eine flächenförmige, in wässriger Umgebung rasch zerfallende oder sich rasch auflösende Darreichungsform mit Räumen oder Hohlräumen in einer polymeren Matrix der Darreichungsform vorgeschlagen, wobei sich der Inhalt der Räume/Hohlräume hinsichtlich seines Aggregatzustands von dem der Matrix unterscheidet.

Allerdings zeigten Untersuchungen, dass empfindliche Personen auch bei der Einnahme einer flächenförmigen Darreichungsform gemäß der Lehre der WO 02/02085 ein unangenehmes oder störendes Empfinden im Mundraum wahrnehmen. In Bezug auf das Auflöseverhalten bei Filmen, die auf Grund der für eine wirksame Dosis erforderlichen Wirkstoffmenge ein hohes Flächengewicht aufweisen müssen, besteht daher ein Bedarf für eine verbesserte Darreichungsform, insbesondere eines Wafers, die bzw. der nicht zu einem unangenehmen Mundempfinden infolge der Auflösung der Darreichungsform, insbesondere des Wafers, führt.

Die vorliegende Erfindung befasst sich mit diesem Bedarf.

### Detaillierte Beschreibung der Erfindung

Zur Lösung der vorstehend geschilderten Aufgabe schlägt die vorliegende Erfindung gemäß Anspruch 1 eine flächenförmige, in wässriger Umgebung sich auflösende oder zersetzende Darreichungsform in Form eines Films oder Wafers zur Freisetzung mindestens eines Wirkstoffs in eine Körperöffnung oder Körperhöhlung vor, die eine Polymermatrix in Form eines Hohlräume aufweisenden verfestigten Schaums sowie mindestens einen pharmazeutischen Wirkstoff umfasst, wobei die Darreichungsform ein Flächengewicht im Bereich von 130 bis 350 g/m² aufweist. Die erfindungsgemäße Darreichungsform weist demzufolge räumliche Bereiche auf, die mit einem Gas wie insbesondere Luft oder Stickstoff gefüllt sind, und die der Darreichungsform ein beschleunigtes Auflöseverhalten vermitteln. Die Hohlräume können sich nur im Innern der Polymermatrix befinden, sie können sich jedoch auch bis zum äußeren Rand der Darreichungsform erstrecken.

Durch die erfindungsgemäßen Hohlräume und die damit verbundene größere Oberfläche der Filme wird insbesondere der Zutritt von Wasser bzw. Speichel oder anderen Körperflüssigkeiten in das Innere der Darreichungsform erleichtert und somit die Auflösung der Darreichungsform und die Wirkstofffreisetzung soweit beschleunigt, dass sich gegebenenfalls nicht gut lösliche Wirkstoffpartikel im Mund und Rachen schnell verteilen können und so nicht mehr wahrnehmbar sind. Dadurch wird ein Verbleiben solcher Partikel an Ort und Stelle verhindert und ein freigesetzter Wirkstoff kann schnell abgeschluckt werden. Daraus resultiert für die erfindungsgemäße Darreichungsform ein verbessertes "Mundgefühl", welches letztendlich zu einer verbesserten Akzeptanz bei den Anwendern oder Patienten führt. Bei einer sublingualen Applikation des Wirkstoffs ermöglicht die erfindungsgemäße Verabreichungsform zudem ein beschleunigtes Bereitstellen des Wirkstoffs zur transmukosalen Aufnahme.

Bei einem schnell resorbierenden Wirkstoff kann zudem durch die schnelle Auflösung der Darreichungsform bei beispielsweiser sublingualer Applikation die transmukosale Resorption verbessert werden. Zum anderen ist die Wandstärke der genannten Hohlräume gering, da diese beispielsweise verfestigte Blasen darstellen, so dass eine schnelle Auflösung oder Zerstörung dieser Hohlräume stattfindet.

Ein weiterer Vorteil der erfindungsgemäßen Darreichungsform besteht darin, dass sich trotz des vergleichsweise hohen Flächengewichts durch die Konfektionierung als Schaum eine schnellere Trocknung realisieren lässt als bei einer vergleichbaren nicht geschäumten Zusammensetzung.

Als bevorzugtes Flächengewicht für die erfindungsgemäße Darreichungsform kann ein Bereich von 130 bis 300 g/m², bevorzugt ein Bereich von 130 bis 250 g/m², weiter bevorzugt ein Bereich von 150 bis 220 g/m² und meist bevorzugt ein Bereich von 165 bis 210 g/m² angegeben werden.

Als Matrixpolymer kommen wasserlösliche Polymere oder Mischungen solcher Polymere zum Einsatz. Dabei werden bevorzugt emulgierende synthetische oder teilsynthetische Polymere oder Biopolymere natürlichen Ursprungs verwendet, die filmbildend und wasserlöslich sind, und/oder die sich zur Schaumbildung eignen. Alternativ können Polymere verwendet werden, die von sich aus nicht emulgierend sind, wenn diese in Kombination mit Tensiden verwendet werden. Geeignete synthetische Polymere sind z.B. Polyvinylalkohol, Polyacrylate und Polyvinylpyrrolidon. Von diesen ist Polyvinylalkohol besonders geeignet. Ein ganz besonders geeigneter Polyvinylalkohol weist ein gewichtsmittleres Molekulargewicht im Bereich von 15 000 bis 60 000 und insbesondere im Bereich von 25 000 bis 50 000 auf. Ein Beispiel für einen geeigneten kommerziellen Polyvinylalkohol ist Mowiol 4-88, der z.B. von Sigma Aldrich vertrieben wird.

Neben den vorstehend genannten Homopolymeren können auch Copolymere als synthetische Polymere zum Einsatz kommen. Geeignete Copolymere sind z.B Polyvinylalkohol-Polyethylenglycol-Propfcopolymere, wie diejenigen, die unter dem Handelsnamen Kollicoat^{®} IR von BASF, oder Polyvinylpyrrolidon-Polyvinylacetatcopolymere, wie diejenigen, die unter dem Handelsnamen Kollidon VA 64 von BASF, erhältlich sind.

Geeignete teilsynthetische Polymere sind Cellulosederivate wie Hydroxypropylmethylcellulose, Carboxymethylcellulose, Hydroxypropylcellulose, Hydroxymethylcellulose und Methylcellulose, sowie andere substituierte CelluloseDerivate. Ein besonders geeignetes Cellulosederivat ist Hydroxypropylmethylcellulose, insbesondere Hydroxypropylmethylcellulose mit einem Methoxysubstitutionsgrad von etwa 28 bis 30 % und einem Hydroxypropoxysubstitutionsgrad von etwa 7 bis 12%. Eine solche Hydroxypropylmethylcellulose ist beispielsweise unter dem Handelsnamen Methocel E von Dow Chemical erhältlich. Ebenfalls bevorzugt sind wasserlösliche Polysaccharide, die pflanzlichen, mikrobiellen oder synthetischen Ursprungs sind, insbesondere solche Polysaccharide, die keine Cellulosederivate sind, wie z. B. Pullulan, Xanthan, Alginate, Dextrane, Agar-Agar, Pektine und Carrageen. Ferner sind auch Proteine, vorzugsweise Gelatine oder andere gelbildende Proteine, sowie Proteinhydrolysate, geeignet. Zu den geeigneten Proteinhydrolysaten gehören, unter anderem, Caseinat, Molke und pflanzliche Proteine, Gelatine sowie (Hühner-) Eiweiß und Mischungen davon. Bevorzugte Proteine sind Caseinate, die von sprühgetrockneten Milchprodukten abstammen.

Als im Rahmen der vorliegenden Erfindung besonders bevorzugte Matrixpolymere gelten Polyvinylalkohol, ein Polyvinylalkohol-Polyethylenglycol-Propfcopolymer und Hydroxypropylmethylcellulose. Polyvinylalkohol ist am meisten bevorzugt. Bei dem Polyvinylalkohol handelt es sich bevorzugt um das Hydrolyseprodukt eines Polyvinylacetat-Homopolymers. In diesem können noch Restanteile von vorzugsweise nicht mehr als 20 mol-% und insbesondere nicht mehr als 15 mol-% (bezogen auf die Gesamtmolmenge an Vinylalkohol- und Vinylacetat-Monomeren) an nicht-hydrolysiertem Polyvinylacetat vorhanden sein. Die vorstehend genannten bevorzugten Matrixpolymere weisen den Vorteil auf, dass ein Zusatz von weiteren oberflächenaktiven Stoffen oder Tensiden zur Erzeugung eines verfestigten Schaums nicht erforderlich ist.

Das Matrixpolymer stellt in der erfindungsgemäßen Darreichungsform neben dem pharmazeutischen Wirkstoff einen Hauptbestandteil der Darreichungsform. Als geeigneter Anteil für das Matrixpolymer kann ein Gehalt von 20 bis 65 Gew.-%, insbesondere 30 bis 60 Gew.-% und meist bevorzugt 32 bis 52 Gew.-%, bezogen auf das Trockengewicht, angegeben werden.

Wie vorstehend bereits angedeutet können die Hohlräume in der erfindungsgemäßen Darreichungsform jeweils isoliert voneinander in der Polymermatrix vorliegen, vorzugsweise in Form von verfestigten Blasen.

Gemäß einer anderen Ausführungsform ist vorgesehen, dass die Hohlräume miteinander in Verbindung stehen, vorzugsweise indem sie ein zusammenhängendes, die Matrix durchdringendes Kanalsystem bilden.

Die genannten Hohlräume sind bevorzugt mit Gas oder einem Gasgemisch, insbesondere Luft oder Stickstoff, gefüllt. Daneben kann es aber auch vorteilhaft sein, wenn die Hohlräume andere Gase oder Gasgemische enthalten, die nicht mit übrigen Bestandteilen der Darreichungsform reagieren. Besonders bevorzugte Gase sind Stickstoff, Kohlendioxid und Helium, sowie ein Gemisch dieser Gase oder von mehreren dieser Gase.

Vorzugsweise haben die genannten Hohlräume einen Volumenanteil von 5 bis 98 %, bevorzugt von 50 bis 80 %, bezogen auf das Gesamtvolumen der Darreichungsform. Auf diese Weise wird der beabsichtigte Effekt der beschleunigten Lösung der Darreichungsform in günstiger Weise beeinflusst.

Ein weiterer wichtiger Parameter, der die Eigenschaften der erfindungsgemäßen Darreichungsform beeinflusst, ist der Durchmesser der Hohlräume oder Blasen. Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann. So kann der Durchmesser der Blasen oder Hohlräume im Bereich von 0,01 bis 60 µm liegen. Besonders bevorzugt liegt der Durchmesser im Bereich von 10 und 50 µm.

Die Oberfläche der Darreichungsform kann eben sein, es ist aber auch möglich, dass die Oberfläche uneben oder unregelmäßig geformt ist, wie beispielsweise wellenförmig oder reliefartig. Eine solche unregelmäßige Oberflächenstruktur kann beispielsweise durch die in die Polymermatrix eingebrachten blasenförmigen Hohlräume verursacht werden, und/oder durch eine nachfolgende TrocknungsBehandlung.

Die erfindungsgemäßen Darreichungsformen sind vorzugsweise dünn, z. B. in Form eines Wafers, gestaltet. Die Dicke der Darreichungsform liegt vorzugsweise zwischen 100 µm bis 5 mm, besonders bevorzugt zwischen 0,5 bis 3 mm.

Hinsichtlich des pharmazeutischen Wirkstoffs unterliegt die vorliegende Erfindung keinen relevanten Beschränkungen, mit der Ausnahme, dass eine orale, wie eine transmucosale, sublinguale, oder gingivale, und/oder gastrointestinale Resorption des Wirkstoffs möglich sein muss.

Geeignet als Wirkstoffe sind demzufolge unter anderem Mittel zur Infektionsbehandlung; Virostatika; Analgetika wie Fentanyl, Sufentanil, Buprenorphin; Anesthetika; Anorectika; Wirkstoffe zur Behandlung von Arthritis und Asthma, wie Terbutalin; Anticonvulsiva; Antidepressiva; Antidiabetika; Antihistaminika; Antidiarrhoika; Mittel gegen Migräne, Juckreiz, Übelkeit und Brechreiz; Reise- bzw. Seekrankheit, wie Scopolamin und Ondansetron; Parkinsonmittel; Antipsychotika; Antipyretika, Spasmolytika, Anticholinergika, Mittel gegen Ulkus wie Ranitidine, Sympathomimetika; Kalziumkanalblocker wie Nifedipin; Betablocker; Beta-Agonisten wie Dobutamin; Antiarrythmika; Antihypertonika wie Atenolol; ACE-Hemmer wie Enalapril; Benzodiazepin-Agonisten wie Flumazenil; coronare, periphere und zerebrale Vasodilatatoren; Stimulation für das Zentralnervensystem; Hormone; Hypnotika; Immunsuppresiva; muskelrelaxierende Mittel; N-Methyl-D-Aspartat (NMDA)-Rezeptor-Antagonisten; Parasympatholytika; Parasympathomimetika; Prostaglandine; Proteine, Peptide; Psychostimulantien; Sedativa; Tranquilizer; Adrenalin.

Im Rahmen der vorliegenden Erfindung besonders bevorzugte Wirkstoffe sind N-Methyl-D-Aspartat (NMDA)-Rezeptor-Antagonisten, insbesondere in Form von Dextromethorphan oder Ketamin oder einem pharmazeutisch wirksamen Derivat davon. Das Ketamin kann als Racemat eingesetzt werden, bevorzugt ist es jedoch, wenn das Ketamin als S-Ketamin in die erfindungsgemäße Darreichungsform einbezogen wird. Geeignete pharmazeutisch wirksamen Derivate von Ketamin sind z.B. nor-S-Ketamin, S-Dehydronorketamin oder das (S,S)-6-Hydroxynorketamin. Die Verwendung von pharmazeutisch akzeptablen Salzen der genannten Wirkstoffe ist ebenfalls von der vorliegenden Erfindung umfasst.

Der Wirkstoffgehalt pro Dosiereinheit beträgt bis zu 100 mg, vorzugsweise bis 50 mg, besonders bevorzugt bis 30 mg und meist bevorzugt bis 20 mg. Auf der anderen Seite sollte der Mindestwirkstoffgehalt pro Dosiereinheit vorzugsweise 5 mg, weiter bevorzugt 10 mg und meist bevorzugt 12 mg betragen. Je nach Anwendungsfall kann die Wirkstoffmenge auch im oberen Bereich der vorstehenden Angaben liegen, beispielsweise im Bereich von mehr als 50 bis 100 mg oder 30 bis 50 mg.

Die auf die Fläche der Darreichungsform relativierte Wirkstoffmenge liegt zweckmäßig im Bereich von 1 bis 15 mg/cm² und bevorzugt 2,8 bis 10 mg/cm².

Der Wirkstoffgehalt in der erfindungsgemäßen Darreichungsform kann innerhalb relativ weiten Grenzen variieren. Als geeignet kann ein Gehaltsbereich von 20 bis 60 Gew.-%, bezogen auf das Trockengewicht der Darreichungsform, angegeben werden. In einer Ausführungsform liegt der Anteil an Wirkstoff in der Darreichungsform eher im unteren Bereich, z.B. wenn der Wirkstoff einen stark unangenehmen Geschmack aufweist, der mit einer größeren Menge an geschmacksmaskierendem Mitteln kompensiert werden muss. In diesem Fall kann als geeigneter Wirkstoffanteil ein Bereich von 21 bis 30 Gew.-% und insbesondere 22 bis 28 Gew.-% angegeben werden. In einer anderen Ausführungsform liegt der Anteil an Wirkstoff in der erfindungsgemäßen Darreichungsform eher im oberen Bereich, wobei ein Gehalt von 42 bis 55 Gew.-% und insbesondere ein Gehalt von 45 bis 52 Gew.-% als besonders bevorzugt angegeben werden kann.

Neben dem pharmazeutischen Wirkstoff kann die erfindungsgemäße Darreichungsform noch weitere Zusatzstoffe enthalten um z.B. das farbliche oder geschmackliche Empfinden beim Einnehmen der Darreichungsform zu beeinflussen.

Ein in diesem Zusammenhang besonders geeigneter Zusatzstoff ist ein geschmacksmaskierendes Mittel, das zu einem verbesserten Geschmackempfinden bei der Einnahme von beispielsweise bitter schmeckenden Wirkstoffen beiträgt. Ein bevorzugtes geschmacksmaskierendes Mittel ist ein Ionenaustauscherharz.

Ionenaustauscherharze, die zur Verwendung in der erfindungsgemäßen Darreichungsform bevorzugt sind, sind wasserunlöslich und bestehen aus einer pharmakologisch inerten organischen oder anorganischen Matrix, die kovalent gebundene funktionelle Gruppen enthält, die ionisch sind oder unter den geeigneten Bedingungen des pH-Werts ionisiert werden können. Die organische Matrix kann synthetisch sein (z. B. Polymere oder Copolymere von Acrylsäure, Methacrylsäure, sulfoniertes Styrol, sulfoniertes Divinylbenzol) oder teilweise synthetisch (z. B. modifizierte Cellulose und Dextrane). Die Matrix kann auch anorganisch sein, z.B. Kieselgel, modifiziert durch Zugabe von ionischen Gruppen. Die kovalent gebundenen Ionengruppen können stark sauer sein (z.B. Sulfonsäure), schwach sauer (z.B. Carbonsäure), stark basisch (z.B. quartäres Ammonium), schwach basisch (z.B. primäres Amin) oder eine Kombination von sauren und basischen Gruppen. Im Allgemeinen sind jene Arten von Ionenaustauschern, die zur Verwendung bei der Ionenaustauschchromatographie und für Anwendungen, wie die Deionisierung von Wasser, geeignet sind, zur Verwendung in den erfindungsgemäßen Darreichungsformen geeignet.

Bei dem Ionentauscherharz handelt es sich bevorzugt um ein Harz auf Basis von vernetztem Polystyrol. Das Polystyrol wird mit einem Vernetzungsmittel vernetzt, das aus difunktionellen Verbindungen ausgewählt ist, die in der Lage sind, Polystyrole zu vernetzen. Vorzugsweise ist das Vernetzungsmittel eine Divinyl- oder Polyvinylverbindung. Am meisten bevorzugt ist das Vernetzungsmittel Divinylbenzol.

Generell ist das Polystyrol zweckmäßig in einem Ausmaß von etwa 3 bis etwa 20%, vorzugsweise etwa 4 bis etwa 16%, bevorzugter etwa 6 bis etwa 10% und am meisten bevorzugt etwa 8 Gew.-%, bezogen auf das gesamte Polystyrol, vernetzt. Das Polystyrol wird mit dem Vernetzungsmittel durch bekannte Mittel vernetzt.

Im Rahmen der vorliegenden Erfindung als geschmacksmaskierende Mittel besonders geeignete Ionentauscherharze haben Austauschkapazitäten unter etwa 6 Milliäquivalenten pro Gramm (meq/g) und vorzugsweise unter etwa 5,5 meq/g.

Die Größe der Ionenaustauscherharzpartikel sollte vorzugsweise in den Bereich von etwa 20 bis etwa 200 Mikrometer fallen. Partikelgrößen deutlich unterhalb der unteren Grenze sind in allen Schritten der Verarbeitung schwer zu handhaben. Teilchengrößen im Wesentlichen oberhalb der Obergrenze, z.B. handelsübliche Ionenaustauscherharze mit sphärischer Form und Durchmessern bis zu etwa 1000 Mikrometern sind in flüssigen Dosierungsformen kiesig und haben eine stärkere Tendenz zum Bruch, wenn sie Trockenhydratisierungszyklen ausgesetzt werden.

Repräsentative Harze, die in dieser Erfindung verwendbar sind, umfassen AMBERLITE IRP-69 (erhältlich von Dow Chemical) und Dow XYS-40010. 00 (erhältlich von der Dow Chemical Company). Beide sind sulfonierte Polymere aus Polystyrol, vernetzt mit 8% Divinylbenzol, mit einer Ionenaustauschkapazität von etwa 4.5 bis 5.5 meq/g Trockenharz (H + Form). Ihr wesentlicher Unterschied liegt in ihrer physischen Form. AMBERLITE IRP-69 umfasst unregelmäßig geformte Partikel mit einem Größenbereich von 47 bis 149 Mikrometer, hergestellt durch Fräsen der übergeordneten, großflächigen Kugeln von AMBERLITE IRP-120. Das Dow XYS-40010. 00-Produkt umfasst kugelförmige Partikel mit einem Größenbereich von 45 bis 150 Mikrometern. Ein weiteres nützliches Austauschharz, Dow XYS-40013. 00, ist ein Polymer, das aus Polystyrol besteht, das mit 8% Divinylbenzol vernetzt ist und mit einer quaternären Ammoniumgruppe funktionalisiert ist. Seine Austauschkapazität liegt normalerweise im Bereich von etwa 3 bis 4 meq/g trockenem Harz. Ein weiteres geeignetes Harz ist AMBERLITE IRP-64.

In weniger bevorzugten Ausführungsformen braucht das geschmacksmaskierende Mittel jedoch kein Ionenaustauscherharz zu sein. In diesen Ausführungsformen kann das geschmacksmaskierende Mittel Magnesiumtrisilicat oder ein Polymer wie EUDRAGIT E (Evonik) und/oder Cellulose, wie Ethylcellulose und dergleichen sein. Der Gehalt an geschmacksmaskierendem Mittel, das in die erfindungsgemäße Darreichungsform einbezogen wird, richtet sich danach, ob der pharmazeutische Wirkstoff einen unangenehmen, z.B. bitteren Geschmack, aufweist. In der Regel bewegt sich der Gehalt an geschmacksmaskierendem Mittel im Bereich von 0,3 bis 45 Gew.-% und insbesondere im Bereich von 0,5 bis 27 Gew.-%, jeweils bezogen auf das Trockengewicht der Darreichungsform.

Zur Modifizierung des Geschmacksempfindens kann die erfindungsgemäße Darreichungsform neben einem geschmacksmaskierenden Mittel oder alternativ dazu ein Süßungsmittel enthalten. Geeignete synthetische Süßungsmittel sind z.B. Sucralose, Aspartam, Cyclamat, Saccharin, Neohesperidin, Thaumatin, Stevia und Acesulfam, sowie deren Salze.

Die erfindungsgemäße Darreichungsform kann weiterhin eine oder mehrere Aromastoffe, ätherische Öle oder Menthol enthalten.

Bei der Herstellung der erfindungsgemäßen Darreichungsform können außerdem eine oder mehrere Säuren beigemischt werden, um dem Schaum eine angenehme saure Geschmacksnote zu verleihen. Beispiele für derartige Säuren schließen, unter anderem, Citronensäure, Milchsäure, Essigsäure, Benzoesäure, Propionsäure, Oxalsäure, Malonsäure, Bernsteinsäure, Äpfelsäure und Weinsäure mit ein. Der Zusatz von Säure(n) kann außerdem notwendig oder erwünscht sein, um den pH-Wert des Schaums abzusenken. Dies ist insbesondere dann erwünscht, wenn der in der Darreichungsform enthaltene Wirkstoff unter basischen Bedingungen relativ unlöslich ist, oder bei Wirkstoffen, die unter basischen Bedingungen nicht stabil sind.

Ferner können der erfindungsgemäßen Darreichungsform, insbesondere Schäume, Anfeuchtungs- bzw. Feuchthaltemittel und/oder Weichmacher zugesetzt werden, um die ästhetischen Eigenschaften des getrockneten Schaums zu verbessern und um die Brüchigkeit oder Sprödigkeit des getrockneten Schaums zu vermindern. Beispiele für solche Mittel sind, unter anderem, Glycerin, Propylenglykol und Polyglycerin-Ester. Der Gehalt an Weichmacher variiert zweckmäßig im Bereich von 2 bis 10 Gew.-%, insbesondere 3 bis 8 Gew.-% und meist bevorzugt 4 bis 6 Gew.-%, bezogen auf das Trockengewicht der Darreichungsform.

In einer Ausführungsform können dem Matrix-Polymer bzw. der Polymermatrix zur Schaumbildung oder dem erhaltenen Schaum vor oder nach dem Trocknen oberflächenaktive Stoffe bzw. Tenside hinzugefügt werden, um die Stabilität des Schaums vor oder nach dem Trocknen zu verbessern. Als Beispiele für geeignete oberflächenaktive Stoffe kommen, unter anderem, substituierte Sorbitanderivate in Betracht, insbesondere diejenigen aus der "Tween"-Serie (ICI) oder "Span"-Serie (TCI). Oberflächenaktive Stoffe können auch in Form von schaumstabilisierenden Polymeren (z.B. Zellulose basierten Polymeren oder Polyacrylat basierten Polymeren, die nicht als Matrixpolymere vorstehend aufgeführt sind), oder in Form von Casein oder Gelatine vorliegen.

Der Anteil des Tensids in der erfindungsgemäßen Darreichungsform hängt in erster Linie davon ab, ob das Matrixpolymer ein Tensid für die Stabilisierung eines Schaums erfordert, was z.B. bei emulgierenden Matrix-Polymeren wie Polyvinylalkohol nicht der Fall ist. Als geeigneter Tensidanteil für die erfindungsgemäßen Darreichungsformen kann ein Bereich von 0 bis 15 Gew.-%, bezogen auf das Trockengewicht der Darreichungsform, angegeben werden.

In einer Ausführungsform erfordert das Matrixpolymer keinen Oberflächenaktiven Stoff oder Tensid für die Stabilisierung eines Schaums. In diesem Fall beträgt der Anteil an Oberflächenaktiven Stoffen und/oder Tensid in der erfindungsgemäßen Darreichungsform vorzugsweise weniger als 0,5 Gew.-% und besonders bevorzugt weniger als 0,1 Gew.-%, bezogen auf das Trockengewicht der Darreichungsform. Es sei hierzu angemerkt, dass die vorstehend als Matrixpolymere aufgeführten Substanzen in Kontext dieser Erfindung nicht als Oberflächenaktive Stoffe angesehen werden, selbst wenn einige dieser Substanzen Oberflächenaktive Eigenschaften aufweisen. Dies ist sogar von Vorteil, da in diesem Fall die vorstehend aufgeführten Oberflächenaktiven Stoffe oder Tenside nicht in die Schaumzusammensetzung einbezogen werden müssen.

Um der Darreichungsform eine gewünschte Farbe zu geben, kann ein Farbstoff oder Farbmittel in die Darreichungsform einbezogen werden. Geeignete Farbstoffe sind z.B. Azofarbstoffe wie Allurarot AC, das auch unter der Handelsbezeichnung FD&C Red 40 erhältlich ist.

Als weitere Zusatzstoffe kommen, unter anderem, Stoffe aus der folgenden Gruppe in Betracht: Carboxymethylcellulose, Gummi arabicum, Methylcellulose, Pektine, modifizierte und nicht modifizierte Stärken, Gelatine, tierische und/oder pflanzliche Proteine, Hühnereiweiß, Alginate, Brij (ein Emulgator), Ethylcitrat, Octylgallat, 1,2-Propylenglycat, Magnesiumstearat, Stearinsäure, mikrokristalline Cellulose, Aerosil, Lecithin, Tween, Propylgallat, Amylogam.

Zusätzlich kann in dem Schaum ein Zucker (oder eine Mischung von Zuckern), oder ein anderes Kohlenhydrat-Material gelöst werden. Der Zucker oder das Kohlenhydrat erhöht die Masse, welche der Schaum nach dem Trocknen hat. Außerdem verleiht das Trocknen und die Kristallisation des Zuckers oder eines anderen Kohlenhydrates, dem getrockneten Schaum eine zusätzliche Festigkeit und Stabilität. Der Zucker oder andere Kohlenhydrate können bei dem getrockneten Schaum eine süße Geschmacksempfindung bewirken oder auf sonstige Weise die organoleptischen Eigenschaften des Schaums verbessern. Beispiele für hierfür verwendbare Zucker sind, unter anderem, Maltose, Lactose, Saccharose, Dextrose (Glucose) und Trehalose, sowie Zuckeralkohole, wie z. B. Mannit, Sorbit, Xylit, Maltit und dergleichen. Als Beispiele für andere Kohlenhydrate kommen Maltodextrin, Stärkezuckersirup (aus Mais), lösliche Stärken und dergleichen in Betracht.

Der Gehalt an Zusatzstoffen sollte, soweit für diese im Vorstehenden keine spezifischen Gehaltsvorgaben gemacht wurden, im Bereich von 0,01 bis 10 Gew.-% und insbesondere 0,1 bis 8 Gew.-%, bezogen auf das Trockengewicht der Darreichungsform, liegen.

Zusätzlich zu den vorgenannten Bestandteilen kann die erfindungsgemäße Darreichungsform Feuchtigkeit (Wasser) enthalten. Als geeigneter Feuchtigkeitsgehalt ein Anteil im Bereich von 2 bis 15 Gew.-% und insbesondere 5 bis 12 Gew.-% angegeben werden.

Die Erfindung ist in erster Line zur Verwendung als orale Darreichungsform, welche Wirkstoffe im Bereich der Mundhöhle freisetzt, vorgesehen. Sie kann aber auch als Darreichungsform, die in andere Körperhöhlungen oder Körperöffnungen eingebracht wird und dort ihre Wirkstoffe freisetzt, verwendet werden. In Frage kommen hier beispielsweise rektale, vaginale oder intranasale Darreichungsformen.

Der aus der Darreichungsform freigesetzte Wirkstoff wird entweder am Applikationsort resorbiert, z. B. über die Mundschleimhaut, oder er wird weitertransportiert und an einem anderen Ort resorbiert (z. B. nach Verschlucken des in der Mundhöhle freigesetzten Wirkstoffs im Magen-Darm-Trakt).

Die Verweildauer der erfindungsgemäßen Darreichungsform am Applikationsort (z. B. Mundraum) bzw. die Zerfallszeit liegt vorzugsweise im Bereich von 1 s bis 5 min, stärker bevorzugt im Bereich von 2 s bis 1 min, weiter bevorzugt im Bereich von 3 bis 10 s und am meisten bevorzugt im Bereich von 3 bis 5 s.

Enthält die Darreichungsform als Wirkstoff Ketamin oder S-Ketamin, so kann diese zweckmäßig zur Behandlung von Leiden eingesetzt werden, bei denen Ketamin oder S-Ketamin zu einer Linderung beitragen. Ein weiterer Aspekt der vorliegenden Erfindung betrifft daher eine Darreichungsform gemäß den vorstehenden Angaben mit einem Gehalt an Ketamin oder S-Ketamin oder pharmazeutisch akzeptablen Salzen davon zur therapeutischen oder prophylaktischen Behandlung von Schmerzen, bevorzugt von chronischen Schmerzen, und weiter bevorzugt von Schmerzen ausgewählt aus der Gruppe umfassend chronische Durchbruchschmerzen, komplexes regionales Schmerzsyndrom, resistente Tumorschmerzen, neuropathische Schmerzen, posttraumatische Syndromschmerzen, ischämische Gliederschmerzen und akute Schmerzen. Alternativ betrifft die vorliegende Erfindung eine Darreichungsform gemäß den vorstehenden Angaben mit einem Gehalt an Ketamin oder S-Ketamin oder pharmazeutisch akzeptablen Salzen davon zur therapeutischen oder prophylaktischen Behandlung von Depressionen. Bei diesen Darreichungsformen ist eine sublinguale Applikation besonders bevorzugt, da dies eine schnelle Verfügbarkeit von Ketamin zur transmukosalen Aufnahme gewährleistet.

Enthält die Darreichungsform als Wirkstoff Dextromethorphan, so kann diese zweckmäßig zur Behandlung von Leiden eingesetzt werden, bei denen Dextromethorphan zu einer Linderung beiträgt. Ein weiterer Aspekt der vorliegenden Erfindung betrifft daher eine Darreichungsform gemäß den vorstehenden Angaben mit einem Gehalt an Dextromethorphan oder einem pharmazeutisch akzeptablen Salz davon zur therapeutischen oder prophylaktischen Behandlung von Husten, Störungen der Gefühlsregulation oder der amyotrophen Lateralsklerose.

Enthält die Darreichungsform als Wirkstoff Adrenalin, so kann diese zweckmäßig zur Behandlung von Leiden eingesetzt werden, bei denen Adrenalin zu einer Linderung beiträgt. Ein weiterer Aspekt der vorliegenden Erfindung betrifft daher eine Darreichungsform gemäß den vorstehenden Angaben mit einem Gehalt an Adrenalin oder einem pharmazeutisch akzeptablen Salz davon zur therapeutischen Behandlung von anaphylaktischem Schock.

Die erfindungsgemäßen Darreichungsformen lassen sich zweckmäßig mit Hilfe der nachstehend angegebenen Verfahren herstellen.

Es wird zunächst eine Lösung oder Dispersion hergestellt, welche mindestens ein wasserlösliches filmbildendes Polymer sowie mindestens einen Wirkstoff enthält. Diese Lösung oder Dispersion, welche auch eine konzentrierte Lösung oder viskose Masse sein kann, wird anschließend durch Eintragen von Gas oder einem Gasgemisch (z. B. Luft) aufgeschäumt. Dies kann mittels eines Dispergierwerks oder einer Schaumaufschlagmaschine geschehen, aber auch durch andere Methoden, z. B. mittels Ultraschall. Als Gase eignen sich insbesondere auch inerte Gase, wie Stickstoff, Kohlendioxid oder Helium, oder Mischungen davon.

Um die so erzeugten Schäume oder luftblasenhaltigen (bzw. gasblasenhaltigen) Massen zu stabilisieren, kann vor oder während der Schaumerzeugung ein schaumstabilisierendes Mittel zugesetzt werden. Hierfür geeignete Mittel, z. B. Tenside, sind dem Fachmann bekannt. Schließlich wird die luftblasenhaltige Masse oder der Schaum auf einer geeigneten Unterlage als Film oder Schicht ausgestrichen und nachfolgend getrocknet.

Das "Trocknen" ist so zu verstehen, dass der Darreichungsform Lösungsmittel, insbesondere Wasser, entzogen wird. Dabei ist es nicht erforderlich, dass der Darreichungsform sämtlichen Lösungsmittel wie Wasser entzogen wird, sondern es ist vielmehr ausreichend, wenn der Darreichungsform der wesentliche Anteil an Lösungsmittel entzogen wird, so dass sich der Schaum verfestigt. Die Darreichungsform kann nach der Trocknung daher einen Restwasseranteil aufweisen, wie er vorstehend für die erfindungsgemäße Darreichungsform angegeben ist.

Durch Lösungsmittel-Entzug verfestigt sich der Schaum während der Trocknung, wobei die gebildeten Hohlräume eine dauerhafte Struktur erhalten. Wafer mit gewünschten Flächenausmassen oder geometrischen Formen werden erhalten, indem die geschäumte Beschichtungsmasse vor der Trocknung in entsprechende Formen gegossen wird, oder indem die einzelnen Wafer aus einem größeren Flächenstück ausgestanzt werden.

Die so erhaltenen wirkstoffhaltigen Arzneiformen weisen die erfindungsgemäßen Eigenschaften und Vorzüge auf.

Die Form, Anzahl und Größe der erzeugten Hohlräume lässt sich mittels verschiedener Verfahrensparameter beeinflussen, z. B. durch die Art und Konzentration der Polymere, durch die Viskosität der Polymermasse, durch Steuerung des Aufschäumungsvorgangs, durch Auswahl der schaumstabilisierenden Mittel etc..

Alternativ zu dem vorstehend beschriebenen Verfahren ist es möglich, die erfindungsgemäßen Darreichungsformen über ein Verfahren herzustellen, bei dem die Ausbildung der Hohlräume im Inneren der Polymermatrix durch Einbringen eines hydrophoben, mit dem für die Herstellung der genannten Lösung oder Dispersion verwendeten Lösungsmittel nicht mischbaren Lösungsmittels erfolgt.

Dabei wird eine Emulsion erzeugt, welche das hydrophobe Lösungsmittel in Form fein verteilter Tröpfchen enthält.

Durch das Entfernen der Lösungsmittel während der nachfolgenden Trocknung bleiben in der Polymermatrix tröpfchen- oder blasenförmige Hohlräume zurück. Bei einem Zwei-Phasen System muss das Lösungsmittel der inneren Phase zuerst entzogen werden.

Ferner kann in Abwandlung des oben beschriebenen Verfahrens die Erzeugung der genannten Hohlräume auf die Weise geschehen, dass der polymer-und wirkstoffhaltigen Lösung oder Dispersion Hilfsstoffe zugesetzt werden, die ein Gas oder Gase bilden, wodurch die Masse aufgeschäumt wird. Dieses Aufschäumen durch Gasentwicklung kann entweder während der Herstellung der Polymermasse oder während der Beschichtung dieser Masse auf die Unterlage erfolgen, oder erst während des nachfolgenden Trocknungsprozesses. Zur Gasbildung geeignete Stoffe oder Stoffgemische sind dem Fachmann bekannt. Das Aufschäumen kann ferner auch durch Entspannung eines vorher gelösten Gases bewirkt werden. Als Gas kann insbesondere ein inertes Gas, wie Stickstoff, Kohlendioxid oder Helium, oder eine Mischung davon verwendet werden.

Bei der Herstellung der erfindungsgemäßen Darreichungsformen kann alternativ auch von einer Schmelze des Matrixpolymers bzw. Polymergemisches ausgegangen werden. Die Verarbeitung erfolgt grundsätzlich ähnlich wie bei im Stand der Technik bekannten Heißschmelz ("hot melt")-Beschichtungsmassen.

In die genannte Polymerschmelze wird durch eine der vorstehend genannten Methoden Gas oder ein Gasgemisch eingetragen, um ein Aufschäumen der Schmelze zu bewirken. Anschließend wird die Schmelze auf eine geeignete Unterlage ausgestrichen oder extrudiert oder in eine Form gegossen, und dann abkühlen bzw. erstarren lassen. Eine Verarbeitung aus der Schmelze kommt nicht in Frage, wenn der vorgesehene Wirkstoff bei der Schmelztemperatur der Polymerschmelze instabil oder flüchtig ist. Falls erforderlich, können der Polymerschmelze Hilfsstoffe zur Herabsetzung des Schmelzpunktes beigefügt werden. Grundsätzlich können auch aus dem Stand der Technik bekannte Heißschmelz-Beschichtungsmassen verwendet werden, sofern diese die in Anspruch 1 genannten Bedingungen erfüllen.

Gemäß einer weiteren Abwandlung der vorstehend beschriebenen Herstellungsverfahren wird die Polymermatrix zunächst in Form eines Blockes hergestellt. Von diesem wird nachfolgend, d. h. nach erfolgter Trocknung bzw. Erstarrung, durch Zerschneiden die gewünschte Darreichungsform abgetrennt.

Die erfindungsgemäße Darreichungsform eignet sich in vorteilhafter Weise für die Verabreichung von Medikamenten in der Mundhöhle oder zur rektalen, vaginalen oder intranasalen Verabreichung. Sie können in der Humanmedizin wie auch in der Veterinärmedizin eingesetzt werden.

### Herstellungsbeispiel

### Beispiel 1

Ein geschäumter Wafer mit Dextromethorphan wurde mit der folgenden Zusammensetzung hergestellt:

| | |
|---|---|
| Polyvinylalcohol (Mowiol 4-88) | 38,00 Gew.-% |
| Roter Farbstoff | 0,20 Gew.-% |
| Saccharin Natrium (Süßstoff) | 1,0 Gew.-% |
| Sucralose (Süßstoff) | 2,0 Gew.-% |
| Glycerin (Weichmacher) | 4,5 Gew.-% |
| Aromastoffe | 6,0 Gew.-% |
| Wirkstoff | 19,7 Gew.-% |
| Geschmacksmaskierendes Mittel | 28,6 Gew.-% |

Für die Herstellung eines verfestigten Schaums wurde zunächst eine Vorlösung des Polyvinylacetats in Wasser hergestellt, in die der Wirkstoff dispergiert wurde. Anschließend wurde die Dispersion unter Lufteintrag aufgeschäumt und mit Hilfe eines Rakels/Streichkastens auf eine Polyethylenträgerfolie aufgebracht. Der Schaum wurde danach im Trockenschrank für 15 Min bei etwa 70 °C unter Erhalt eines verfestigten Schaums getrocknet. Der so hergestellte Schaum wies ein Flächengewicht von 200 g/m² auf.

### Beispiel 2

Die in der nachstehenden Tabelle 1 angegebene Zusammensetzung wurde gemäß der in Beispiel 1 angegebenen Vorschrift unter Verwendung von Ketamin HCl als Wirkstoff zu einem verfestigten Schaum verarbeitet. Parallel dazu wurde die Zusammensetzung ohne Schäumen zu einem Vergleichsfilm verarbeitet.

**Tabelle 1:**

| | |
|---|---|
| | 1 |
| Wirkstoff [g] | 50 |
| Polyvinylalkohol (Mowiol 4-88) [g] | 38,3 |
| Roter Farbstoff [g] | 0,2 |
| Saccharin Natrium [g] | 1,0 |
| Sucralose [g] | 2,0 |
| Glycerin [g] | 4,5 |
| Aromastoffe [g] | 3,5 |
| Geschmacksmaskierendes Mittel [g] | 0,5 |
| Flächengewicht [g/m²] | 200 |

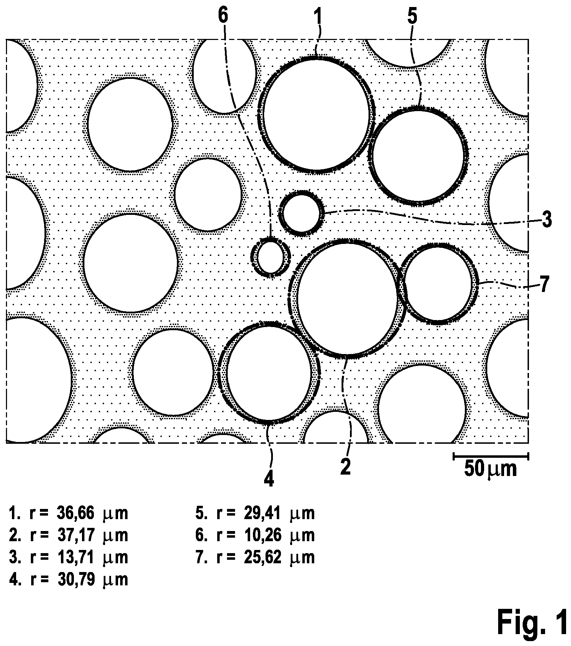

Der verfestigte Schaum wurde mikroskopisch untersucht. Ein mikroskopische Aufnahme ist in Figur 1 wiedergegeben, die zeigt dass die Filme Hohlräume mit Durchmessern im Bereich von etwa 10 bis 37 µm 50 µm. Insgesamt konnten in dem Film Hohlraum Größen im Bereich von 10 bis 50 µm nachgewiesen werden.

Die Disintegration der hergestellten Schäume und Vergleichsfilme wurde bestimmt. Dabei wurden für die Schäume Disintegrationszeiten von etwa 13 Sekunden bestimmt (Mittelwert aus 6 Messungen). Die Vergleichsfilme wiesen deutlich langsamere Disintegrationszeiten von etwa 38 Sekunden auf.

## Patentansprüche

1. Flächenförmige, in wässriger Umgebung sich auflösende oder zersetzende Darreichungsform in Form eines Films oder Wafers zur Freisetzung mindestens eines Wirkstoffs in eine Körperöffnung oder Körperhöhlung, umfassend eine Polymermatrix in Form eines Hohlräume aufweisenden verfestigten Schaums sowie mindestens einen pharmazeutischen Wirkstoff, **dadurch gekennzeichnet, dass** die Darreichungsform ein Flächengewicht im Bereich von 130 bis 350 g/m² aufweist.

2. Darreichungsform nach Anspruch 1, **dadurch gekennzeichnet, dass** diese ein Flächengewicht im Bereich von 130 bis 300 g/m², bevorzugt 130 bis 250 g/m², weiter bevorzugt im Bereich von 150 bis 220 g/m² und noch weiter bevorzugt im Bereich von 165 bis 210 g/m² aufweist.

3. Darreichungsform nach Anspruch 1 oder 2, **dadurch gekennzeichnet, dass** die Polymermatrix auf einem Polymer ausgewählt aus der Gruppe umfassend Polyvinylalkohol, ein Polyvinylalkohol-Polyethylenglycol-Propfcopolymer und Hydroxypropylmethylcellulose beruht.

4. Darreichungsform nach einem der Ansprüche 1 bis 3, **dadurch gekennzeichnet, dass** die Hohlräume voneinander isoliert sind und vorzugsweise in Gestalt von Blasen vorliegen.

5. Darreichungsform nach einem der Ansprüche 1 bis 3, **dadurch gekennzeichnet, dass** die Hohlräume miteinander in Verbindung stehen und vorzugsweise ein die Polymermatrix durchdringendes Kanalsystem bilden.

6. Darreichungsform nach einem der Ansprüche 1 bis 5, **dadurch gekennzeichnet, dass** die Hohlräume mit Luft oder einem Gas, vorzugsweise mit einem inerten Gas, besonders bevorzugt mit Stickstoff, Kohlendioxid, Helium, einem Gemisch dieser Gase oder einem Gemisch von mehreren dieser Gase, gefüllt sind.

7. Darreichungsform nach einem der Ansprüche 1 bis 6, **dadurch gekennzeichnet, dass** die genannten Hohlräume einen Volumenanteil von 5 bis 98 %, bevorzugt von 50 bis 80 %, bezogen auf das Gesamtvolumen der Darreichungsform, ausmachen.

8. Darreichungsform nach einem der vorangehenden Ansprüche, **dadurch gekennzeichnet, dass** die Darreichungsform als Wafer gestaltet ist, wobei die Dicke der Darreichungsform vorzugsweise zwischen 100 µm bis 5 mm, besonders bevorzugt zwischen 0,5 bis 3 mm liegt.

9. Darreichungsform nach einem der vorangehenden Ansprüche, **dadurch gekennzeichnet, dass** der pharmazeutische Wirkstoff Ketamin, bevorzugt in Form von S-Ketamin, oder Dextromethorphan oder ein pharmazeutisch akzeptables Salz davon enthält.

10. Darreichungsform nach einem der vorangehenden Ansprüche, **dadurch gekennzeichnet, dass** der pharmazeutische Wirkstoff einen Anteil im Bereich von 38 bis 60 Gew.-%, bevorzugt von 42 bis 55 Gew.-% und besonders bevorzugt von 45 bis 52 Gew.-%, bezogen auf das Gesamtgewicht der Darreichungsform, in dieser ausmacht.

11. Darreichungsform nach einem der vorangehenden Ansprüche, **dadurch gekennzeichnet, dass** die Darreichungsform einen geschmackmaskierenden Bestandteil, bevorzugt in Form eines Ionentauscherharzes, enthält.

12. Verfahren zur Herstellung einer Darreichungsform nach einem der Ansprüche 1 bis 11, **gekennzeichnet durch** das
- Herstellen einer Lösung oder Dispersion, die zumindest ein Matrixpolymer und mindestens einen pharmazeutischen Wirkstoff enthält;
- Aufschäumen der Lösung oder Dispersion durch Eintragen eines Gases oder Gasgemisches, oder durch chemische Gaserzeugung, oder durch Entspannen eines gelösten Gases, gegebenenfalls nach vorherigem Zusatz eines schaumstabilisierenden Mittels;
- Ausstreichen der aufgeschäumten Lösung oder Dispersion auf eine Beschichtungsunterlage; und
- Verfestigen der ausgestrichenen Lösung oder Dispersion durch Trocknen und Entzug des Lösungsmittels.

13. Verfahren zur Herstellung einer Darreichungsform nach einem der Ansprüche 1 bis 11, **gekennzeichnet durch** das
a) Herstellen einer Lösung oder Dispersion, die zumindest ein Matrixpolymer und mindestens einen pharmazeutischen Wirkstoff enthält;
b) Zusetzen eines Hilfsstoffes oder einer Kombination von Hilfsstoffen, welche zur Gasbildung befähigt sind;
c) Ausstreichen der Lösung oder Dispersion auf eine Beschichtungsunterlage; und
d) Verfestigen der ausgestrichenen Lösung oder Dispersion durch Trocknen und Entzug des Lösungsmittels.

14. Verfahren zur Herstellung einer Darreichungsform nach einem der Ansprüche 1 bis 11, **gekennzeichnet durch** das
a) Herstellen einer polymerhaltigen Schmelze (hot melt), welche mindestens ein Matrixpolymer sowie mindestens einen pharmazeutischen Wirkstoff enthält;
b) Aufschäumen der Schmelze durch Eintragen eines Gases oder Gasgemisches, oder durch chemische Gaserzeugung, oder durch Entspannen eines gelösten Gases, gegebenenfalls nach vorherigem Zusatz eines schaumstabilisierenden Mittels;
c) Ausstreichen der Schmelze auf eine Beschichtungsunterlage; und
d) Verfestigung des Filmes durch Abkühlung.

15. Verfahren nach einem der Ansprüche 12-14, **dadurch gekennzeichnet, dass** die Schritte c) und d) durch folgende Schritte e) und f) ersetzt oder modifiziert werden:
e) Herstellen der Polymermatrix in Form eines Blockes, ausgehend von der Lösung oder Dispersion bzw. von der Schmelze;
f) Zerschneiden des verfestigten Blocks, um flächenförmige Formen zu erhalten.

16. Darreichungsform nach einem der Ansprüche 1 bis 11 oder Verfahrensprodukt eines Verfahrens gemäß einem der Ansprüche 14 bis 16 zur Verwendung zur Verabreichung von pharmazeutischen Wirkstoffen in der Mundhöhle.

17. Darreichungsform nach einem der Ansprüche 1 bis 11 oder Verfahrensprodukt eines Verfahrens gemäß einem der Ansprüche 12 bis 15 zur Verwendung zur rektalen, vaginalen oder intranasalen Verabreichung von pharmazeutischen Wirkstoffen.

18. Darreichungsform nach Anspruch 9 oder einem davon abhängigen Anspruch mit einem Gehalt an Ketamin, S-Ketamin oder einem pharmazeutisch akzeptablen Salz davon zur therapeutischen oder prophylaktischen Behandlung von Schmerzen, bevorzugt von chronischen Schmerzen, oder Depressionen.

19. Darreichungsform nach Anspruch 9 oder einem davon abhängigen Anspruch mit einem Gehalt an Dextromethorphan oder einem pharmazeutisch akzeptablen Salz davon zur therapeutischen oder prophylaktischen Behandlung von Husten, Störungen der Gefühlsregulation oder der amyotrophen Lateralsklerose.

## Claims

1. A planar dosage form in the form of a film or wafer that disintegrates or dissolves in an aqueous environment for releasing at least one active ingredient in a body orifice or body cavity, comprising a polymer matrix in the form of a solidified foam having cavities, and at least one pharmaceutical active ingredient, **characterised in that** the dosage form has a mass per unit area in the range of 130 to 350 g/m².

2. The dosage form according to claim 1, **characterised in that** it has a mass per unit area in the range of 130 to 300 g/m², preferably 130 to 250 g/m², more preferably in the range of 150 to 220 g/m², and even more preferably in the range of 165 to 210 g/m².

3. The dosage form according to claim 1 or claim 2, **characterised in that** the polymer matrix is based on a polymer selected from the group comprising polyvinyl alcohol, a polyvinyl alcohol-polyethylene glycol graft copolymer, and hydroxypropyl methylcellulose.

4. The dosage form according to any one of claims 1 to 3, **characterised in that** the cavities are isolated from one another and are preferably present in the form of bubbles.

5. The dosage form according to any one of clams 1 to 3, **characterised in that** the cavities are connected to one another and preferably form a channel system penetrating the polymer matrix.

6. The dosage form according to any one of claims 1 to 5, **characterised in that** the cavities are filled with air or a gas, preferably with an inert gas, especially preferably with nitrogen, carbon dioxide, helium, a mixture of these gases, or a mixture of a plurality of these gases.

7. The dosage form according to any one of claims 1 to 6, **characterised in that** said cavities have a volume fraction of 5 to 98%, preferably of 50 to 80%, based on the total volume of the dosage form.

8. The dosage form according to any one of the preceding claims, **characterised in that** the dosage form is formed as a wafer, wherein the thickness of the dosage form is preferably between 100 µm and 5 mm, especially preferably between 0.5 and 3 mm.

9. The dosage form according to any one of the preceding claims, **characterised in that** the pharmaceutical active ingredient contains ketamine, preferably in the form of S-ketamine, or dextromethorphan, or a pharmaceutically acceptable salt thereof.

10. The dosage form according to any one of the preceding claims, **characterised in that** the pharmaceutical active ingredient accounts for a proportion in the range of 38 to 60 % by weight, preferably of 42 to 55 % by weight, and especially preferably of 45 to 52 % by weight, based on the total weight of the dosage form.

11. The dosage form according to any one of the preceding claims, **characterised in that** the dosage form contains a taste-masking constituent, preferably in the form of an ion exchange resin.

12. A method for producing a dosage form according to any one of claims 1 to 11, **characterised by** the
- production of a solution or dispersion which contains at least one matrix polymer and at least one pharmaceutical active ingredient;
- foaming of the solution or dispersion by introduction of a gas or gas mixture, or by chemical gas generation, or by expansion of a dissolved gas, optionally after prior addition of a foam-stabilising agent;
- spreading of the foamed solution or dispersion onto a coating substrate; and
- solidification of the spread solution or dispersion by drying and removal of the solvent.

13. A method for producing a dosage form according to any one of claims 1 to 11, **characterised by** the
a) production of a solution or dispersion which contains at least one matrix polymer and at least one pharmaceutical active ingredient;
b) addition of an auxiliary or a combination of auxiliaries which are capable of gas formation;
c) spreading of the solution or dispersion onto a coating substrate; and
d) solidification of the spread solution or dispersion by drying and removal of the solvent.

14. A method for producing a dosage form according to any one of claims 1 to 11, **characterised by** the
a) production of a polymer-containing melt (hot melt) which contains at least one matrix polymer and at least one pharmaceutical active ingredient;
b) foaming of the melt by introducing a gas or gas mixture, or by chemical gas generation, or by expansion of a dissolved gas, optionally after prior addition of a foam-stabilising agent;
c) spreading of the melt onto a coating substrate; and
d) solidification of the film by cooling.

15. The method according to any one of claims 12-14, **characterised in that** steps c) and d) are replaced or modified by the following steps e) and f):
e) production of the polymer matrix in the form of a block starting from the solution or dispersion or from the melt;
f) cutting of the solidified block in order to obtain planar forms.

16. Dosage form according to any one of claims 1 to 11 or a product of a method according to any one of claims 14 to 16 for the use in administering pharmaceutical active ingredients in the oral cavity.

17. Dosage form according to one of claims 1 to 11 or a product of a method according to any one of claims 12 to 15 for the use in rectal, vaginal or intranasal administration of pharmaceutical active ingredients.

18. The dosage form according to claim 9 or a clam dependent thereon with a content of ketamine, S-ketamine or a pharmaceutically acceptable salt thereof for the therapeutic or prophylactic treatment of pain, preferably of chronic pain or depression.

19. The dosage form according to claim 9 or a claim dependent thereon with a content of dextromethorphan or a pharmaceutically acceptable salt thereof for the therapeutic or prophylactic treatment of coughs, emotional dysregulation disorders, or amyotrophic lateral sclerosis.

## Revendications

1. Forme posologique plate, se dissolvant ou se décomposant dans un environnement aqueux sous la forme d'un film ou d'une pastille, destinée à libérer au moins un principe actif dans un orifice corporel ou une cavité corporelle, comprenant une matrice polymère sous la forme d'une mousse solidifiée présentant des cavités ainsi qu'au moins un principe actif pharmaceutique, **caractérisée en ce que** la forme posologique présente un poids surfacique dans la plage de 130 à 350 g/m².

2. Forme posologique selon la revendication 1, **caractérisée en ce que** celle-ci présente un poids surfacique dans la plage de 130 à 300 g/m², de manière préférée de 130 à 250 g/m², de manière davantage préférée dans la plage de 150 à 220 g/m² et de manière encore davantage préférée dans la plage de 165 à 210 g/m².

3. Forme posologique selon la revendication 1 ou 2, **caractérisée en ce que** la matrice polymère repose sur un polymère choisi parmi le groupe comprenant de l'alcool polyvinylique, un copolymère de greffage de polyéthylène glycol-alcool polyvinylique et de l'hydroxypropylméthylcellulose.

4. Forme posologique selon l'une quelconque des revendications 1 à 3, **caractérisée en ce que** les cavités sont isolées les unes des autres et sont présentes de préférence sous la forme de bulles.

5. Forme posologique selon l'une quelconque des revendications 1 à 3, **caractérisée en ce que** les cavités sont reliées entre elles et forment de préférence un système de canaux traversant la matrice polymère.

6. Forme posologique selon l'une quelconque des revendications 1 à 5, **caractérisée en ce que** les cavités sont remplies d'air ou d'un gaz, de préférence d'un gaz inerte, de manière particulièrement préférée d'azote, de dioxyde de carbone, d'hélium, d'un mélange desdits gaz ou d'un mélange de plusieurs desdits gaz.

7. Forme posologique selon l'une quelconque des revendications 1 à 6, **caractérisée en ce que** lesdites cavités représentent une fraction volumique de 5 à 98 %, de manière préférée de 50 à 80 %, par rapport au volume total de la forme posologique.

8. Forme posologique selon l'une quelconque des revendications précédentes, **caractérisée en ce que** la forme posologique est configurée en tant que pastille, dans laquelle l'épaisseur de la forme posologique se situe de préférence entre 100 µm à 5 mm, de manière particulièrement préférée entre 0,5 à 3 mm.

9. Forme posologique selon l'une quelconque des revendications précédentes, **caractérisée en ce que** le principe actif pharmaceutique contient de la kétamine, de manière préférée sous la forme de S-kétamine, ou du dextrométhorphane ou un sel pharmaceutiquement acceptable.

10. Forme posologique selon l'une quelconque des revendications précédentes, **caractérisée en ce que** le principe actif pharmaceutique représente une fraction dans la plage de 38 à 60 % en poids, de manière préférée de 42 à 55 % en poids, et de manière particulièrement préférée de 45 à 52 % en poids, par rapport au poids total de la forme posologique dans celle-ci.

11. Forme posologique selon l'une quelconque des revendications précédentes, **caractérisée en ce que** la forme posologique contient un élément constitutif masquant le goût, de manière préférée sous la forme d'une résine à échange d'ions.

12. Procédé de fabrication d'une forme posologique selon l'une quelconque des revendications 1 à 11, **caractérisé par**
- la fabrication d'une solution ou dispersion, qui contient au moins un polymère matriciel et au moins un principe actif pharmaceutique ;
- le moussage de la solution ou de la dispersion par apport d'un gaz ou d'un mélange de gaz, ou par production chimique de gaz, ou par détente d'un gaz dissous, éventuellement après ajout préalable d'un agent de stabilisation de mousse ;
- l'étalement de la solution ou dispersion moussée sur une sous-couche de revêtement ; et
- la solidification de la solution ou de la dispersion étalée par séchage et retrait du solvant.

13. Procédé de fabrication d'une forme posologique selon l'une quelconque des revendications 1 à 11, **caractérisé par**
a) la fabrication d'une solution ou d'une dispersion, qui contient au moins un polymère matriciel et au moins un principe actif pharmaceutique ;
b) l'ajout d'un adjuvant ou d'une combinaison d'adjuvants, lesquels sont capables de former un gaz ;
c) l'étalement de la solution ou de la dispersion sur une sous-couche de revêtement ; et
d) la solidification de la solution ou de la dispersion étalée par séchage et retrait du solvant.

14. Procédé de fabrication d'une forme posologique selon l'une quelconque des revendications 1 à 11, **caractérisé par**
a) la fabrication d'une matière fondue contenant du polymère (fondue à chaud), laquelle contient au moins un polymère matriciel ainsi qu'au moins un principe actif pharmaceutique ;
b) le moussage de la matière fondue par apport d'un gaz ou d'un mélange de gaz ou par production chimique de gaz, ou par détente d'un gaz dissous, éventuellement après l'ajout préalable d'un agent de stabilisation de mousse ;
c) l'étalement de la matière fondue sur une sous-couche de revêtement ; et
d) la solidification du film par refroidissement.

15. Procédé selon l'une quelconque des revendications 12
- 14, **caractérisé en ce que** les étapes c) et d) sont remplacées ou sont modifiées par des étapes e) et f) qui suivent :
e) la fabrication de la matrice polymère sous la forme d'un bloc en partant de la solution ou de la dispersion ou de la matière fondue ;
f) la découpe du bloc solidifié pour obtenir des formes plates.

16. Forme posologique selon l'une quelconque des revendications 1 à 11 ou produit de procédé d'un procédé selon l'une quelconque des revendications 14 à 16 pour l'utilisation pour administrer des principes actifs pharmaceutiques dans la cavité buccale.

17. Forme posologique selon l'une quelconque des revendications 1 à 11 ou produit de procédé d'un procédé selon l'une quelconque des revendications 12 à 15 pour l'utilisation pour administrer par voie rectale, vaginale ou intranasale des principes actifs pharmaceutiques.

18. Forme posologique selon la revendication 9 ou une revendication dépendant de celle-ci avec une teneur en kétamine, en S-kétamine ou en un sel pharmaceutiquement acceptable de celle-ci pour le traitement thérapeutique ou prophylactique de douleurs, de manière préférée de douleurs chroniques, ou de dépressions.

19. Forme posologique selon la revendication 9 ou selon une revendication dépendant de celle-ci avec une teneur en dextrométhorphane ou en un sel pharmaceutiquement acceptable de celui-ci pour le traitement thérapeutique ou prophylactique de toux, de troubles de la régulation de l'humeur ou de la sclérose latérale amyotrophique.
